# EUROPEAN PATENT SPECIFICATION

(11) **EP 2 974 723 B1**
(45) Date of publication and mention of the grant of the patent: **11.04.2018**
(21) Application number: 14764587.3
(22) Date of filing: 13.02.2014
(51) Int. Cl.: A61K 31/365, A61K 31/192, A61K 31/235, A61P 21/04, A61P 25/14, A61K 9/00, A61K 31/7048

(54) **APPLICATION OF PHTHALIDE COMPOUNDS**
ANWENDUNG VON PHTHALID-VERBINDUNGEN
APPLICATION DE COMPOSÉS PHTALIDES

(30) Priority: 12.03.2013 US 201361777127 P
(43) Date of publication of application: 20.01.2016
(73) Proprietor: Hawking Biological Technology Co., Ltd., New Taipei City (TW)
(72) Inventor: LIN, Shinn-zong, New Taipei City Taiwan (TW); HARN, Horng-jyh, New Taipei City Taiwan (TW); CHIOU, Tzyy-wen, New Taipei City Taiwan (TW); HSUEH, Kuo-wei, New Taipei City Taiwan (TW); HUANG, Mao-hsuan, New Taipei City Taiwan (TW)
(74) Representative: dompatent von Kreisler Selting Werner - Partnerschaft von Patent- und Rechtsanwälten mbB
(86) International application number: PCT/CN2014/000155
(87) International publication number: WO 2014/139318

(56) References cited:
- CN-A- 102 397 272
- US-A1- 2009 176 873
- US-A1- 2011 177 106
- US-A1- 2012 020 930
- LIU, XIAOLI ET AL.: 'Experimental Study on Effects of Butyl Phthalide on Ischemic Tolerance Induced by Focal Cerebral Preconditioning' CHINESE JOURNAL OF GERIATRICS vol. 31, no. 6, 30 June 2012, XP008181002

## Description

### Field of the Invention

The present invention relates to the use of a phthalide, especially to the use of a phthalide in the manufacture of a medicament for increasing the expression level of at least one of telomerase, brain-derived neurotrophic factor (BDNF), stromal cell-derived factor-1 (SDF1), CXC chemokine receptor 4 (CXCR4), and an immune regulatory factor in a stem cell, in particular the use of a phthalide in the manufacture of a medicament for treating motor neuron degenerative diseases and/or delaying the onset of motor neuron degenerative diseases.

### Descriptions of the Related Art

A neuron, also known as a nerve cell, is one of the structural and functional units of the nervous system of an organism. Neurons can transmit messages to other cells by chemical and electrical signals. Neurons can vary in shape and size, and the diameters of neurons may range from about 4 µm to about 100 µm. The structure of a neuron can be roughly divided into three parts: a cell body, dendrites, and an axon, wherein the dendrites can transmit signals into the cell body, and the axon can transmit signals out from the cell body.

Neurons can be classified into three types depending on their functions and signal transmission directions: sensory neurons, motor neurons and interneurons, wherein a motor neuron is a nerve cell controlling the body activities of an organism. In general, motor neurons in the brain are known as upper motor neurons, while motor neurons in the brain stem and the spinal cord are known as lower motor neurons. Malfunctions caused by the degeneration of motor neurons may result in motor neuron degenerative diseases, such as amyotrophic lateral sclerosis (ALS), myasthenia gravis, myasthenia, muscular atrophy, muscular dystrophy, multiple sclerosis, multiple-system atrophy, and spinal muscular dystrophy. Patients suffering from the aforesaid motor neuron degenerative diseases will gradually show symptoms such as muscle weakness, atrophy, trembling, cramping rigidity, which may lead to such as difficulty speaking, difficulty swallowing, respiratory failure.

The real cause of motor neuron degenerative diseases is still uncertain to date. However, researches showed that the possible causes of the diseases include neuronal death caused by excessive autophagy stimulated by the accumulation of superoxide anions, autoimmune disorder, excessive excitation of neurons (e.g., excessive accumulation of glutamates), excessive oxidation of neuron, heredity, etc. Medicines presently used in clinic to treat motor neuron degenerative diseases include glutamate antagonists such as Riluzole, antioxidants such as vitamin E, neurotrophic factors, immune modulators, etc. However, the aforesaid medicines usually do not have significant therapeutic effects or may only prolong the life of the patients for 3 to 6 months.

In addition, researches found that a stem cell can differentiate into a neural cell, which brings hope for the treatment of nervous system diseases, such as Parkinson's disease, stroke, brain injury, and spinal cord injury. For example, researches found that a stem cell can pass through the blood brain barrier via intravenous injection (i.e., intravenous transplantation) to alleviate neurodegeneration caused by aging and can repair and reconstruct the damaged cerebrovascular to maintain the normalization and/or rejuvenation of cranial nerves. Furthermore, researches revealed that a stem cell therapy has shown evident therapeutic effect on many patients suffering from combined brain atrophy and Alzheimer's disease. However, the aforementioned researches also showed that the stem cell therapy does not have significant therapeutic effect on motor neuron degenerative diseases or may only provide a limited effect, such as prolonging the longevity of the mice suffering from ALS to about 140 days (see such as "Garbuzova-Davis et al., Human umbilical cord blood treatment in a mouse model of ALS: optimization of cell dose, 2008," which is entirely incorporated hereinto by reference). Therefore, there is still an need for a medicament for treating motor neuron degenerative diseases and/or delaying the onset of motor neuron degenerative diseases.

US 2011/0177106 A1 discloses a method for treating a subject to reduce inflammation and/or to modulate an immune response, wherein said method comprises administering, to a subject in need of such treatment, an effective amount of an isolated compound having the following formula: 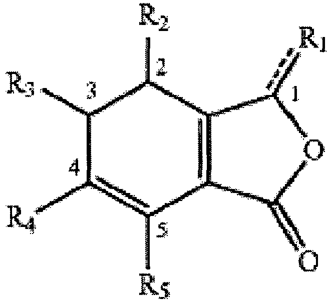 wherein
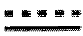 represents a carbon-carbon single bond or a carbon-carbon double bond;
R₁ is alkyl or CR₆, wherein R6 is alkyl, acyl, haloalkyl, alkylamino or hydroxylalkyl;
R₂, R₃ and R₄ are, independently, H, acyl, halo, haloalkyl, amino, alkylamino, hydroxyl, alkyl, hydroxylalkyl, or ---COOH; and
R₅ is ---H, alkyl, halo, haloalkyl, amino, alkylamino,
hydroxylalkyl, or ---COOH.

US 2009/0176873 A1 discloses a method for the control or treatment of conditions requiring modulation of inflammation in a mammal which comprises administering to a mammal in need of such treat-ment an effective amount of a compound represented by formula (I) 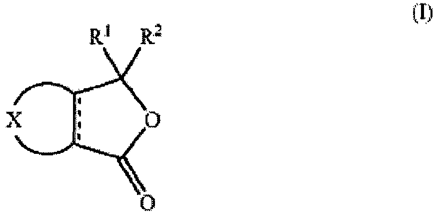 wherein
the dotted line is an optional bond;
R¹ is butyl or butyryl if R² is hydroxyl but is butyl if R² is hydrogen; or R¹ and R² taken together are 1-butylidene optionally substituted by hydroxyl, methyl, or 3-(a,(3-dimethylacrylyloxy)-pentylidenyl;
X is a residue selected from the group consisting of X1, X2, X3, X4, and X5; 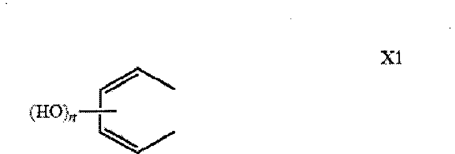 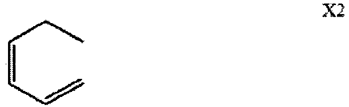 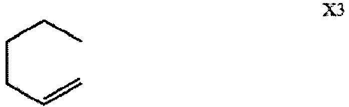 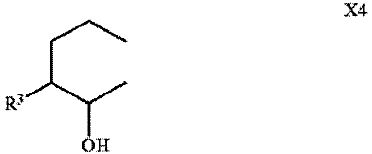 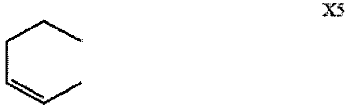 wherein
X is X2, X3 or X5 if the dotted line in formula (I) is absent;
and X is X1, X4 or X5 if the dotted line signifies a bond in formula (I) above;
R³ is hydroxyl or butyryl; and
n is 1 or 2.

US 2012/0020930 A1 discloses A method for the preparation of adipose stem cells comprising of exposing said adipose derived stem cells to a CD26 antagonist or inhibitor.

The inventors of the present invention found that phthalide can increase the expression levels of telomerase, brain-derived neurotrophic factor, stromal cell-derived factor-1, CXC chemokine receptor 4, and/or an immune regulatory factor (such as interleukin-6 and interleukin-8) in a stem cell. Furthermore, a combined use of a phthalide and a stem cell can provide an effect on treating and/or delaying the onset of motor neuron degenerative diseases.

### SUMMARY OF THE INVENTION

An objective of the present invention is to provide a use of a phthalide in the manufacture of a medicament for increasing the expression level of at least one of telomerase, brain-derived neurotrophic factor, stromal cell-derived factor-1, CXC chemokine receptor 4, and an immune regulatory factor in a stem cell.

Another objective of the present invention is to provide a kit, comprising: a first composition comprising a phthalide; and a second composition comprising a stem cell, wherein the first composition and the second composition are to be administered to a subject in need simultaneously or separately.

Yet another objective of the present invention is to provide a use of a metabolic precursor of a phthalide in the manufacture of a medicament for treating and/or delaying the onset of a motor neuron degenerative disease, wherein the metabolic precursor is 3-butylidene-4,5-dihydrophthalide (ligustilide).

The detailed technology and the preferred embodiments implemented for the present invention will be described in the following paragraphs for people skilled in the field to well appreciate the features of the claimed invention.

The present invention is reflected in the independent claims. Preferred embodiments are described in the dependent claims.

### BRIEF DESCRIPTION OF THE DRAWINGS

**Fig. 1** shows the metabolism of n-butylidenephthalide (BP) in an organism, wherein **Fig. 1A** is an LC-MS/MS spectrum of the sample of a mixture from the reaction of BP and human hepatic microsomes, **Fig. 1B** is a metabolic profile showing the phase I metabolism of BP in an organism, and **Fig. 1C** is a metabolic profile showing the phase II metabolism of BP in an organism;
**Fig. 2** is a curve diagram showing the survival rate of SOD1-G93A transgenic mice treated with different conditions;
**Fig. 3** is a curve diagram showing the BBB-scaled curves of SOD1-G93A transgenic mice treated with different conditions;
**Fig. 4** is a bar diagram showing the telomerase expression levels of stem cells treated with different ingredients;
**Fig. 5** is an electrophoresis picture showing the gene expression levels of BDNF, SDF1, CXCR4, IL-6 and IL-8 in stem cells treated with different ingredients; and
**Fig. 6** shows the protein expression levels in the SOD1-G93A transgenic mice treated with a combination of BP and stem cells, wherein **Fig. 6A** is a picture showing the immunohistochemical staining performed with an anti-human mitochondria antibody, **Fig. 6B** is a picture showing the immunohistochemical staining performed with an anti-human BDNF antibody, and **Fig. 6C** is a picture showing the immunohistochemical staining performed with an anti-human CXCR4 antibody.

### DETAILED DESCRIPTION OF THE INVENTION

The following will describe some embodiments of the present invention in detail. However, without departing from the spirit of the present invention, the present invention may be embodied in various forms and should not be limited to the embodiments described in the specification. In addition, unless otherwise state herein, the term "a," "the," or the like recited in the specification of the present invention (especially in the claims) should include both the singular and the plural forms. The term "optionally substituted" or "optionally replaced" used in this specification should include both the conditions of being substituted or replaced and not being substituted or replaced. Furthermore, the term "effective amount" used in this specification refers to the amount of the compound that can at least partially alleviate the condition that is being treated in a suspected subject when administered to the subject. The term "subject" used in this specification refers to a mammalian, including human and non-human animals. The term "mg/kg-body weight" used in this specification refers to the dosage (mg) required per kg-body weight. The term "hydrocarbyl" used in this specification refers to a saturated hydrocarbyl or an unsaturated hydrocarbyl with one or more π-bonds. The term "stem cell therapy" used in this specification refers to any therapy comprising administering to a subject a stem cell in any manner.

Telomere is a region of repetitive nucleotide sequences at each end of a chromatid. It has been known that the length of telomere may determine the longevity of a cell. Telomerase is a reverse transcriptase and can extend the length of telomeres, and thus plays an important role in regulating cell growth, maintaining cell homeostasis, and inhibiting cell apoptosis. As described above, previous researches revealed that a stem cell therapy does not have significant therapeutic effect on motor neuron degenerative diseases, which probably due to the short lifecycle of the transplanted stem cells. Therefore, if the lifecycle of the transplanted stem cells can be prolonged (e.g., the activity of telomerase is enhanced), the therapeutic effect of stem cell on motor neuron degenerative diseases can be improved.

Furthermore, in stem cell therapies, it has been known that the brain-derived neurotrophic factor (BDNF), stromal cell-derived factor-1 (SDF1), and CXC chemokine receptor 4 (CXCR4) secreted from stem cells have an effect on inhibiting the apoptosis of motor neurons and promoting the proliferation of motor neurons, and thus, can be used to protect motor neurons (the effects of BDNF and SDF1 on neurons can be seen in such as "Park, D. et al., Human adipose tissue-derived mesenchymal stem cells improve cognitive function and physical activity in ageing mice. J Neurosci Res, 2013," which is entirely incorporated hereinto by reference). In another aspect, it has been known that the immune regulatory factors secreted from stem cells, such as interleukin-6 (IL-6) and interleukin-8 (IL-8), can maintain neurogenesis by regulating immune response (the researches about maintaining neurogenesis by regulating immune responses can be seen in such as "Kohman, R.A. et al., Neurogenesis, inflammation and behavior. Brain Behav Immun, 2013. 27 (1) : p. 22-32," which is entirely incorporated hereinto by reference). Accordingly, if the expression levels of BDNF, SDF1, CXCR4, and/or immune regulatory factors (e.g., IL-6, IL-8) in a stem cell can be increased, the therapeutic effect of the stem cell therapy on motor neuron degenerative diseases can be improved.

The inventors of the present invention found that a phthalide can increase the expression levels of telomerase, BDNF, SDF1, CXCR4, and/or an immune regulatory factor (e.g., IL-6, and/or IL-8) in stem cells.

Accordingly, the present invention provides a use of a phthalide in the manufacture of a medicament for increasing the expression level of at least one of telomerase, BDNF, SDF1, CXCR4, and an immune regulatory factor (such as IL-6 and IL-8) in a stem cell. In accordance with the invention the phthalide is selected from the group: n-butylidenephthalide (BP), 3-butylidene-4,5-dihydrophthalide (ligustilide), and combinations thereof. When the medicament is administered to a subject treated with a stem cell therapy (i.e., a subject administered with a stem cell) or a subject to be treated with a stem cell therapy (i.e., a subject to be administered with a stem cell), the phthalide can increase the expression levels of telomerase, BDNF, SDF1, CXCR4, and/or an immune regulatory factor in a stem cell, and thus, can prolong the lifecycle of the stem cells, inhibit the apoptosis of motor neurons and/or promote the proliferation of motor neurons, and can treat motor neuron degenerative diseases and/or delay the onset of motor neuron degenerative diseases. The phthalide as disclosed herein may be at least one selected from the following group: a compound of formula (I), a pharmaceutically acceptable salt of the compound of formula (I), a pharmaceutically acceptable ester of the compound of formula (I), and combinations thereof: 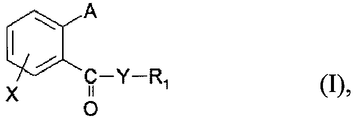 wherein, A is a C1-C5 hydrocarbyl being optionally substituted by one or more substituents 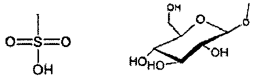 selected from -OH, =O, and C1-C3 hydrocarbyl; X is H, -OH, , or ; R₁ is H or a substituted or unsubstituted C 1-C20 hydrocarbyl, wherein one or more -CH₂- in the hydrocarbyl are optionally replaced by -NH- or -O-; Y is O or S and optionally bonds with A to form a five-membered ring, with a proviso that when Y bonds with A to form a five-membered ring, R₁ is not present.

The phthalide disclosed herein also comprises a compound of formula (I), wherein A is a C1-C5 alkyl or alkenyl being optionally substituted by one or more substituents selected from -OH, =O, and C1-C3 alkyl; and R₁, if present, is H or a substituted or unsubstituted C1-C10 hydrocarbyl, wherein one or more -CH₂- in the hydrocarbyl are optionally replaced by -NH- or -O-. Preferably, in the compound of formula (I), A is 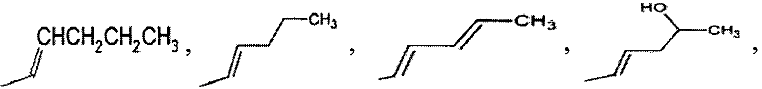 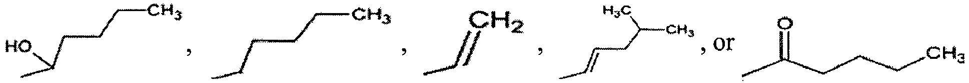 ; and R₁, if present, is H,

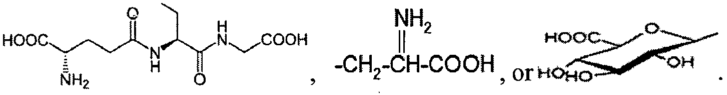

More specifically, the phthalide as disclosed herein may be selected from the group consisting of the following Compounds (1) to (14), a pharmaceutically acceptable salt thereof, a pharmaceutically acceptable ester thereof, and combinations thereof: 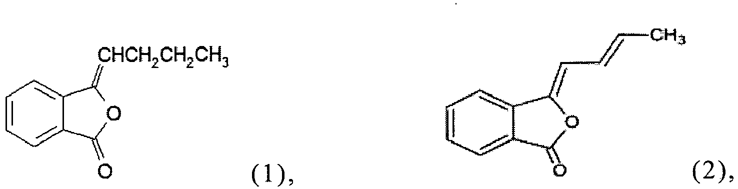 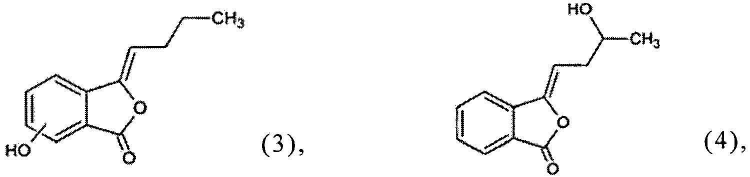 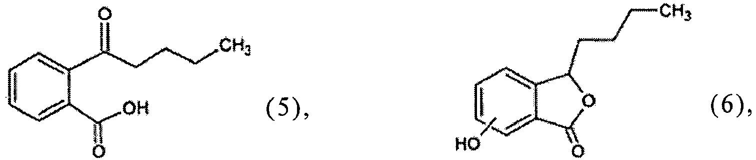 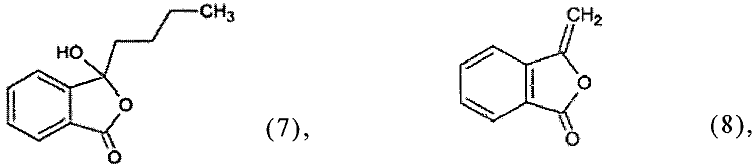 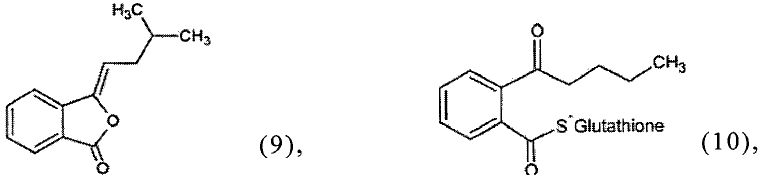 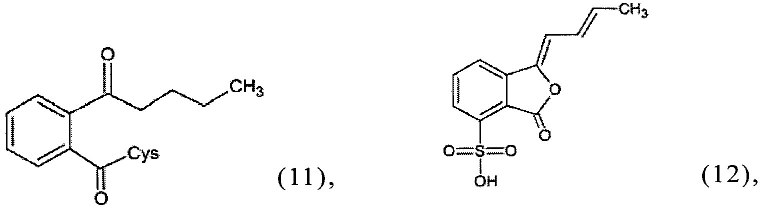 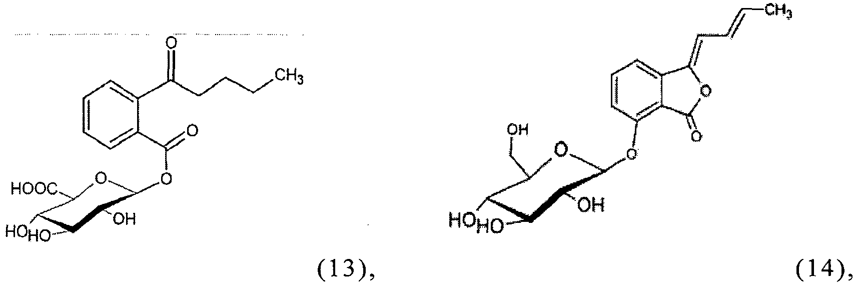 wherein the "Cys" in Compound (10) refers to cysteine. In accordance with the invention the phthalide is selected from the group: n-butylidenephthalide (BP), 3-butylidene-4,5-dihydrophthalide (ligustilide), and combinations thereof. The inventors of the present invention found that n-butylidenephthalide, when administered to an organism, can increase the expression levels of telomerase, BDNF, SDF1, CXCR4, and/or an immune regulatory factor in the stem cells of the organism. In the above Compounds (1) to (14), Compound (1) is n-butylidenephthalide (BP), and Compounds (2) to (14) are metabolites of BP, namely, the compounds generated by the phase I or phase II metabolism of BP in an organism as illustrated by the examples provided hereinafter in this specification. It is believed that the pharmaceutical activity of a compound in an organism could be attributable to the metabolites of the compound. Therefore, it is preferred that the phthalide used in the present invention is selected from the following group: Compounds (1) to (14), pharmaceutically acceptable salts thereof, pharmaceutically acceptable esters thereof, and combinations thereof.

In addition to the compound of formula (I), the phthalide used in the present invention could be another structural analogue of BP, a pharmaceutically acceptable salt thereof, a pharmaceutically acceptable ester thereof, or a combination thereof.

The term "pharmaceutically acceptable salt" used in this specification refers to a pharmaceutically acceptable salt formed by an above-mentioned compound having acid functional group(s) and an organic or inorganic base. The salts formed with inorganic bases include, but are not limited to, alkali metal salts (e.g., sodium salts, potassium salts), alkaline earth metal salts (e.g., calcium salts, magnesium salts), transition metal salts (e.g., iron salts, zinc salts, copper salts, manganese salts, and aluminum salts), and ammonium salts. The salts formed with organic bases include, but are not limited to, the salts formed with methylamine, dimethylamine, trimethylamine, ethylamine, diethylamine, triethylamine, isopropylamine, tripropylamine, tributylamine, ethanolamine, diethanolamine, 2-dimethylaminoethanol, 2-diethylaminoethanol, dicyclohexylamine, lysine, arginine, histidine, caffeine, hydrabamine, choline, betaine, ethylenediamine, glucosamine, methylglucamine, theobromine, purines, piperidine, N-ethylpiperidine, tetramethylammonium compounds, tetraethylammonium compounds, pyridine, N,N-dimethylaniline, N-methylpiperidine, N-methylmorpholine, dicyclohexylamine, dibenzylamine, N,N-dibenzylphenethylamine, 1-ephenamine, N,N'-dibenzylethylenediamine, and polyamine resins.

The term "pharmaceutically acceptable ester" used in this specification includes an ester formed from the reaction of an acid and an above-mentioned compound having such as -OH functional group(s). The acid may be an inorganic acid such as hydrochloric acid, hydrobromic acid, sulfuric acid, sulfamic acid, nitric acid, and phosphoric acid, or an organic acid such as acetic acid, trifluoroacetic acid, adipic acid, ascorbic acid, aspartic acid, benzenesulfonic acid, benzoic acid, butyric acid, camphoric acid, camphorsulfonic acid, cinnamic acid, citric acid, digluconic acid, ethanesulfonic acid, glutamic acid, glycolic acid, glycerophosphoric acid, hemisulfic acid, hexanoic acid, formic acid, 2-hydroxy ethane-sulfonic acid (isethionic acid), lactic acid, hydroxymaleic acid, malic acid, malonic acid, mandelic acid, mesitylenesulfonic acid, methanesulfonic acid, naphthalenesulfonic acid, nicotinic acid, 2-naphthalenesulfonic acid, oxalic acid, pamoic acid, pectinic acid, phenylacetic acid, 3-phenylpropionic acid, pivalic acid, propionic acid, pyruvic acid, salicylic acid, stearic acid, succinic acid, sulfanilic acid, tartaric acid, p-toluenesulfonic acid, and undecanoic acid.

The term "structural analogue of BP" used in this specification refers to a compound which is not identical to BP, but is in part or entirely similar to part or whole of BP in terms of chemical structure, electron distribution and/or pharmaceutical characteristics. In addition to the compound of formula (I), examples of a structural analogue of BP include, but are not limited to, a metabolic precursor of BP, an isomer of BP or an isomer of a metabolic precursor of BP, and a pharmaceutically acceptable salt or ester thereof. The term "metabolic precursor of BP" used in this specification refers to a compound whose metabolism in an organism will generate BP. Specific examples of a structural analogue of BP include, but are not limited to, 3-butylidene-4,5-dihydrophthalide (ligustilide) and 3-butyl-3a,4,5,7a-tetrahydro-1(3H)-isobenzofuranone (cnidilide).

In some embodiments of the present invention, the phthalide used in the present invention is 3-butylidene-4,5-dihydrophthalide.

In the use of the present invention, the stem cell can be any stem cells suitable for a stem cell therapy, such as a stem cell selected from the following group: an embryonic stem cell, an adult stem cell (e.g., a mesenchymal stem cell, a hematopoietic stem cell), an induced pluripotent stem cell (iPSc), and combinations thereof. Examples of the mesenchymal stem cell include, but are not limited to, a bone marrow stem cell, an umbilical cord blood stem cell, a placenta stem cell, an adipose stem cell (ADSC), an oral stem cell, an olfactory bulbs stem cell, an amniotic fluid stem cell, an amniotic. stem cell, an umbilical cord stem cell, and an umbilical cord lining stem cell. In some embodiments of the use of the present invention, the stem cell is an adipose stem cell.

As described above, in the use of the present invention, the medicament as provided is effective in increasing the expression levels of telomerase, BDNF, SDF1, CXCR4, and an immune regulatory factor (e.g., IL-6, IL-8) in a stem cell, and thus, can be used to prevent the apoptosis of motor neurons, protect motor neurons, and/or promote the proliferation of motor neurons, and can be used for treating motor neuron degenerative diseases and/or delaying the onset of motor neuron degenerative diseases.

In some embodiments, the medicament provided by the present invention is used to treat motor neuron degenerative diseases and/or delay the onset of the diseases. The motor neuron degenerative diseases include any diseases related to the degeneration of motor neurons including but not limited to amyotrophic lateral sclerosis (ALS), myasthenia gravis, gravis, muscular atrophy, muscular dystrophy, multiple sclerosis, multiple system atrophy, and spinal muscular atrophy.

According to one embodiment of the present invention, the medicament can be used for treating ALS. It is known that patients suffering from ALS will gradually show muscular atrophy, which usually causes quadriplegia, difficulty swallowing and even respiratory failure in 2 to 5 years from the onset of the disease. Researches showed that ALS may be relevant to excessive excitation of neurons (e.g., excessive accumulation of glutamates). Therefore, at present, the glutamate antagonist, such as Riluzole, is primarily used in clinic to treat ALS to increase the survival rate of the patients. As compared with the administration of Riluzole, the medicament provided by the present invention can more effectively delay the onset of motor neuron degenerative diseases and increase the survival rate of the patients.

In the present invention, the medicament can be provided in any suitable form and be administered by any suitable manner. For example, the medicament can be applied by oral administration, nasal administration, corticospinal tract injection, intrathecal injection, intracerebral injection, intravenous injection, intraperitoneal injection, and/or subcutaneous injection to a subject in need, but is not limited thereby. Because a medicament in a form for oral administration is convenient for the subject to applied by themselves, in one embodiment according to the present invention, the medicament is provided in a form suitable for oral administration, such as a tablet, a capsule, a granule, powder, a fluid extract, a solution, syrup, a suspension, an emulsion or a tincture. Depending on the form and purpose, the medicament may further comprise a pharmaceutically acceptable carrier.

As for the dosage form suitable for oral administration, the medicament can comprise a pharmaceutically acceptable carrier that will not adversely affect the activity of the active component (i.e., the phthalide) comprised therein, such as a solvent, an oily solvent, a diluent, a stabilizer, an absorption retarder, a disintegrant, an emulsifier, an antioxidant, a binder, a lubricant, and a moisture absorbent. The composition can be provided in any suitable form for oral administration in any suitable manner.

As for the dosage form for corticospinal tract injection, intrathecal injection, intracerebral injection, intravenous injection, or subcutaneous injection, the medicament may comprise one or more components such as an isotonic solution, a saline buffer solution (e.g., a phosphate buffer solution or a citrate buffer solution), a solubilizer, an emulsifier, and other carriers to manufacture the medicament as an intravenous injection, an emulsion intravenous injection, a powder injection, a suspension injection, or a powder-suspension injection.

In addition to the above adjuvants, the medicament may optionally comprise other additives, such as a flavoring agent, a toner, a coloring agent, to enhance the taste and visual perception of the resultant medicament. To improve the storability of the resultant medicament, the medicament may also comprise a suitable amount of a preservative, a conservative, an antibacterial agent, an antifungal agent, etc. Furthermore, the medicament provided by the present invention may optionally comprise one or more other active components such as an antioxidant (e.g., vitamin E), a neurotrophic factor, an immune modulator, to further enhance the efficacy of the medicament or to increase the application flexibility and adaptability of the medicament thus provided, as long as the other active component(s) will not affect the desired effect of the phthalide used therein.

To make the medicament provided by the present invention to increase the expression levels of telomerase, BDNF, SDF1, CXCR4, and/or an immune regulatory factor (e.g., IL-6 and IL-8) in a stem cell and thus improve the efficacy of a stem cell therapy on a subject, the medicament can be administered to the subject before, after, or simultaneously with the administration of the stem cell to the subject. In the aforesaid stem cell therapy, the dosage of the stem cells is preferably about 1×10² cells/site of administration to about 1×10¹⁵ cells/site of administration, and more preferably about 1×10⁵ cells/site of administration to about 1×10⁸ cells/site of administration. However, for patients suffering from acute conditions, the dosage of the stem cells can be increased to several times or several tens of times, depending on the actual demand. In addition, depending on the demand of the subject to be administered, the medicament can be applied with various administration frequencies, such as once a day, several times a day or once for days. For example, when applied to a human body for treating motor neuron degenerative diseases and/or delaying the onset of motor neuron degenerative diseases, the dosage of the medicament is preferably about 100 mg (as the phthalide)/kg-body weight to about 1,000 mg (as the phthalide)/kg-body weight per day, and more preferably about 250 mg (as the phthalide)/kg-body weight to about 800 mg (as the phthalide)/kg-body weight per day. Furthermore, depending on the demand, the daily dosage of the medicament can be optionally adjusted to be administered in one or more times, to further improve the therapeutic effect of the phthalide. For example, when the desired dosage of the medicament is 500 mg/kg-body weight per day, the medicament can be administered once at a dosage of 500 mg/kg-body weight or be administered twice a day at a dosage of 250 mg/kg-body weight.

The present invention also provides a kit, comprising: a first composition comprising a phthalide, and a second composition comprising a stem cell, wherein the first composition and the second composition are to be administered to a subject in need simultaneously or separately. In addition, when the kit of the present invention is used, the first composition and the second composition can be optionally applied with the same or different administration frequencies, such as once a day, several times a day or once for days. The properties, features and the preferred embodiments of the phthalide and the stem cells are all in line with the above descriptions.

The kit of the present invention can be used for increasing the expression level of at least one of telomerase, BDNF, SDF1, CXCR 4, and an immune regulatory factor (e.g., IL-6 and IL-8) in a stem cell. Therefore, the kit can be used for preventing the apoptosis of motor neurons, protecting motor neurons, and/or promoting the proliferation of motor neurons, and thus, can be used for treating motor neuron degenerative diseases and/or delaying the onset of the diseases. Preferably, the kit of the present invention is used for treating spinal motor neuron degenerative diseases and/or delaying the onset of the diseases. The motor neuron degenerative diseases include any diseases related to the degeneration of motor neurons such as amyotrophic lateral sclerosis (ALS), myasthenia gravis, gravis, muscular atrophy, muscular dystrophy, multiple sclerosis, multiple system atrophy, and spinal muscular atrophy, but are not limited thereby. According to an embodiment of the present invention, the kit is used for treating ALS.

In the kit of the present invention, the first composition and the second composition are preferably placed in different packages, such as different plastic bags, plastic bottles, glass bottles, ampoules, cartons or plastic boxes. The packages can be connected to or separated from each other. In addition, the first composition and the second composition can be independently provided in any suitable form for administration and in the same form or in different forms. For example, the first composition and the second composition can be independently applied to a subject in need via oral administration, nasal administration, corticospinal tract injection, intrathecal injection, intracerebral injection, intravenous injection, intraperitoneal injection, and/or subcutaneous injection, but is not limited thereby. In some embodiments according to the present invention, the first composition is provided in a dosage form for oral administration (e.g., a tablet), and the second composition is provided in a glass bottle as a dosage form suitable for intracerebral injection or intravenous injection.

Depending on the form and purpose, the first composition and the second composition may further comprise any pharmaceutically acceptable carriers, adjuvants, and additives so as to be provided in a desired form for administration, as long as the carriers, adjuvants, and additives will not adversely affect the desired effect of the phthalide and/or the stem cells.

As for the dosage form suitable for oral administration, the first composition and the second composition can independently comprise a pharmaceutically acceptable carrier which will not adversely affect the desired activity of the active component (i.e., the phthalide or the stem cell) comprised therein, such as a solvent, an oily solvent, a diluent, a stabilizer, an absorption retarder, a disintegrant, an emulsifier, an antioxidant, a binder, a lubricant, and a moisture absorbent. The first composition and/or the second composition can be provided in a form suitable for oral administration in any suitable manner, such as a tablet, a capsule, a granule, powder, a fluid extract, a solution, syrup, a suspension, an emulsion, or a tincture.

As for the dosage form for corticospinal tract injection, intrathecal injection, intracerebral injection, intravenous injection, or subcutaneous injection, the first composition and the second composition may independently comprise one or more components such as an isotonic solution, a saline buffer solution (e.g., a phosphate buffer solution or a citrate buffer solution), a solubilizer, an emulsifier, and other carriers so as to be manufactured as an intravenous injection, an emulsion intravenous injection, a powder injection, a suspension injection, a powder-suspension injection, etc.

In addition to the above adjuvants, the first composition and/or the second composition may optionally comprise other additive(s) such as a flavoring agent, a toner, and a coloring agent to enhance the taste and visual perception of the resultant medicament. To improve the storability of the resultant medicament, the first composition and/or the second composition may also comprise a suitable amount of a preservative, a conservative, an antibacterial agent, and/or an antifungal agent. Furthermore, the first composition and/or the second composition of the kit of the present invention may optionally comprise one or more other active components, to further enhance the efficacy of the medicament provided by the present invention or to increase the application flexibility and adaptability of the resultant formulation, as long as the other active components will not adversely affect the desired effect of the phthalide and/or the stem cell in the kit.

In addition, depending on the actual demand, the dosage of the first composition and the second composition of the kit of the present invention can be adjusted to that suitable for single or multiple use. Therefore, the kit of the present invention may optionally further comprise an instruction to show the manner for using the first composition and the second composition. For example, when the kit of the present invention is applied to a human body for treating motor neuron degenerative diseases and/or delaying the onset of motor neuron degenerative diseases, the dosage of the first composition is preferably about 100 mg (as the phthalide)/kg-body weight to about 1,000 mg (as the phthalide)/kg-body weight per day, and more preferably about 250 mg (as the phthalide)/kg-body weight to about 800 mg (as the phthalide)/kg-body weight per day. The dosage of the second composition is preferably from about 1×10² cells (as the stem cells)/site of administration to about 1×10¹⁵ cells (as the stem cells)/site of administration, and more preferably about 1×10⁵ cells (as the stem cells)/site of administration to about 1×10⁸ cells (as the stem cells)/site of administration. However, for patients suffering from acute conditions, the dosage can be increased to several times or several tens of times, depending on the actual demand. In one embodiment of treating ALS according to the present invention, the phthalide is BP and its dosage is about 500 mg/kg-body weight per day, and the stem cell is adipose stem cell and its dosage is about 2×10⁶ cells once for intracerebral injection and 1×10⁶ cells once for intravenous injection.

The kit of the present invention may further comprise a third composition comprising a solvent and/or a solution. The third composition is preferably placed in a package (e.g., a plastic bag, a plastic bottle, a glass bottle or an ampoule) different from that of the first composition and the second composition, and can be mixed with the first composition and/or the second composition to provide an injection solution. Examples of the solvent suitable for the third composition include, but are not limited to, polar solvents, such as water, DMSO and ethanol. Examples of the solution suitable for the third composition include, but are not limited to, saline buffer solutions and any other injection solutions suitable for providing a form suitable for injection. Examples of the saline buffer solution include, but are not limited to, a phosphate buffer solution (PBS), a citrate buffer solution, and a physiological saline. In one embodiment of the present invention, the second composition (comprising a stem cell) is mixed with the third composition (comprising PBS) to provide a form suitable for injection before being administered.

In addition, the inventors of the present invention found that a metabolic precursor of a phthalide also has the effects on treating a motor neuron degenerative disease and/or delaying the onset of the disease, and the efficacy of the metabolic precursor of a phthalide is not only superior to that of using BP alone, but also superior to that of using BP in combination with a stem cell. Therefore, the present invention also provides a use of a metabolic precursor of a phthalide in the manufacture of a medicament for treating a motor neuron degenerative disease and/or delaying the onset of the disease, wherein the metabolic precursor is 3 -butylidene-4,5 -dihydrophthalide (ligustilide).

According to the present invention, a medicament provided by using a metabolic precursor of a phthalide can be used for treating and/or delaying the onset of the following diseases: amyotrophic lateral sclerosis, myasthenia gravis, myasthenia, muscular atrophy, muscular dystrophy, multiple sclerosis, multiple system atrophy, spinal muscular atrophy, and combinations thereof, and especially can be used in treating ALS and/or delaying the onset of ALS. The dosage form, manner of administration, and dosage of the medicament are all in line with the above descriptions.

The present invention will be further illustrated in details with specific examples as follows. However, the following examples are provided only for illustrating the present invention, and the scope of the present invention is not limited thereby.

### [Examples]

### [Example 1] Metabolites of n-butylidenephthalide (BP)

It has been known that the medicine metabolic pathway within an organism's liver can be primarily divided into phase I and phase II metabolism. Phase I metabolism occurs mainly by the redox reaction or hydrolysis reaction of medicine, while phase II metabolism occurs mainly by cytochrome P450 (CYP450) monoxygenase system. This example simulated the phase I and II metabolism of BP that occur within an organism's liver by respectively mixing BP with hepatic microsomes or cryopreserved hepatocytes *in vitro.* The products in the reaction solution were analyzed by liquid chromatograph-tandem mass spectrometer (LC-MS/MS) to identify the metabolites and the metabolic profile.

### (1) Phase I metabolism assay

BP (comprising Z-BP 95% + E-BP 5%; purchased from ECHO Chemical) (2 µM) was mixed respectively with K₃PO₄ buffer solution (100 mM, pH 7.4) containing human, rats or dogs hepatic micrsomes (0.5 mg/mL). The mixture was maintained at 37°C for 10 minutes, and then pre-warmed cofactors (NADPH (2 mM) and MgCl₂ (3 mM)) were added thereto and the mixture was incubated at 37°C for 60 minutes. Thereafter, 3-fold volume of acetonitrile containing 0.1% formic acid was added to the mixture to terminate the reaction. The mixture was centrifuged at 13000 rpm for 5 minutes. The supernatant was then collected and analyzed by LC-MS/MS to identify the metabolites.

### (2) Phase II metabolism assay

William's E medium containing 5×10⁵ thawed human, rat or dog hepatocytes were respectively added into a 12-well culture dish, and the cells were cultured for 6 hours. Then, 0.5 mL of BP (comprising Z-BP 95% + E-BP 5%; purchased from ECHO Chemical) (50 µM) was added into the culture dish. After the cells were incubated at 37°C, 95% relative humidity and 5% CO₂ for 6 hours, 2 mL of acetonitrile (100%) was added to terminate the reaction. The sample was collected, mixed adequately, and centrifuged at 45000 g, 4°C for 10 minutes. The supernatant was then collected, dried, and analyzed by LC-MS/MS to identify the metabolites of BP.

### (3) LC-MS/MS analysis

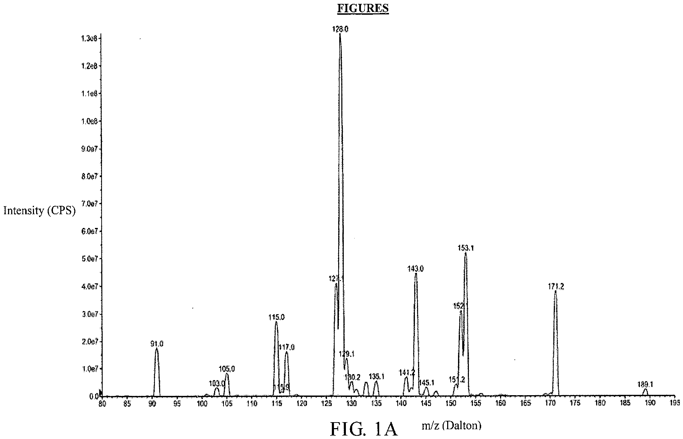

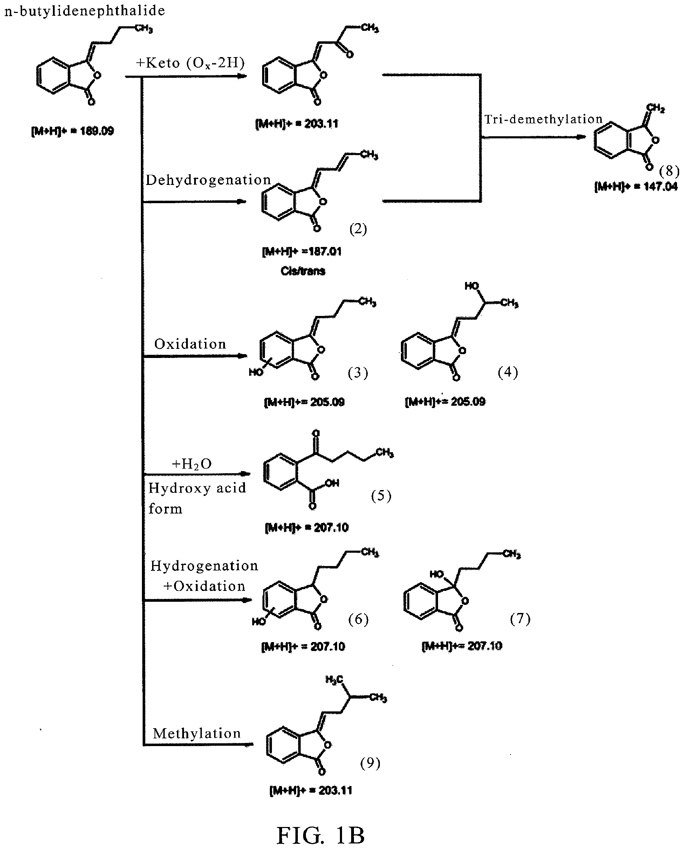

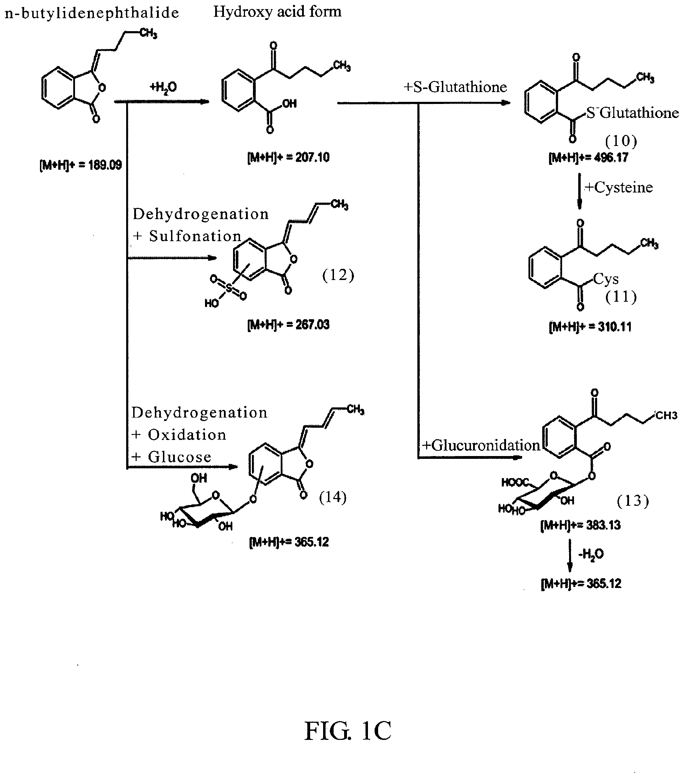

The samples obtained from (1) and (2) were separately dissolved in acetonitrile containing 0.1 % formic acid-, centrifuged at 45000 g, 4°C for 10 minutes. Then, an aliquot of each sample was injected into an autosampler vial (Agilent Technologies, USA) to perform LC-MS/MS analysis. The LC-MS/MS system comprises an ABSCIEX 5500 Q TRAP™ system with 1200SL HPLC system (Agilent Technologies, USA), a HPLC column (Symmetry® C18, 3.5 µM, 4.6 × 75 mm), and an autosampler (Agilent Technologies, USA). The mass spectrometry analysis was performed in positive ion electrospray ionization (+ESI) mode at 5.5 kV, 550°C, and N₂ (nitrogen) was used as an auxiliary gas. A two-solvent system (solvent A: 0.1% formic acid; solvent B: methanol containing 0.1% formic acid-) was used to perform HPLC at a flow rate of 0.8 mL/min. The HPLC gradient system was set as follows: 0 to 2 minutes held at 10% solvent B; 2 to 7 minutes with a gradient from 10% to 95% solvent B; 7 to 12 minutes held at 95% solvent B; 12 to 14 minutes with a gradient from 95% to 10% solvent B; 14 to 20 minutes held at 10% solvent B; and the retention time of HPLC analysis is 20 minutes. The aliquots (20 µl) of each sample was injected into the LC-MS/MS system to perform analysis. The most intense peaks in the LC-MS/MS spectrum and the mass-shifted peaks in the LC-MS/MS spectrum of each sample as compared to BP spectrum were analyzed by LightSight™ Software to determine the metabolites in the samples and identifying the biotransformation pathway and metabolic profile of BP in an organism. The results are shown in Table 1, Table 2, Figure 1A, Figure 1B and Figure 1C.

Figure 1A shows the fragment product spectrum of the mixture of BP (m/z= 189.1) and human hepatic microsomes analyzed by LC-MS/MS. As shown in Figure 1A, the most intense peaks (m/z) are 171.2 amu, 153.1 amu, 143.0 amu, 128.0, and 115.0 amu. Figure 1B shows the metabolic profile obtained from the reaction of the mixture of BP and hepatic microsomes, and the chemical structures of compounds (2) to (9). Figure 1C shows the metabolic profile obtained from the reaction of the mixture of BP and cryopreserved hepatocytes, and the chemical structures of compounds (11) to (14).

Table 1 shows the types of metabolites obtained from the reaction of the mixture of BP and hepatic microsomes (i.e., phase I metabolism) and the biotransformation pathway acquired by software analysis. The results show that the compounds (2) to (9) of the present invention can be produced by the reaction of a mixture of BP and the hepatic microsomes of rat, dog or human, which indicates that BP can be transformed into similar metabolites when metabolized in the livers of different organisms.

**Table 1**

| Phase I metabolites | | | |
|---|---|---|---|
| Species | Metabolites | Biotransformation pathway | Mass-shifted peaks |
| Rat | Compound (2) | Dehydrogenation | m/z 189→187 |
| | Compound (3); Compound (4) | Oxidation | m/z 189→205 |
| | Compound (5); Compound (6); Compound (7) | Hydrogenation (forming hydrocarbyl group) | m/z 189→207 |
| | Compound (8) | Tri-Demethylation | m/z 189→147 |
| | Compound (9) | +Keto (Oₓ-2H) or methylation | m/z 189→203 |
| Dog | Compound (2) | Dehydrogenation | m/z 189→187 |
| | Compound (3); Compound (4) | Oxidation | m/z 189→205 |
| | Compound (5); Compound (6); Compound (7) | Hydrogenation (forming hydrocarbyl group) | m/z 189→207 |
| | Compound (8) | Tri-Demethylation | m/z 189→147 |
| | Compound (9) | +Keto (Oₓ-2H) or methylation | m/z 189→203 |
| Human | Compound (2) | Dehydrogenation | m/z 189→187 |
| | Compound (3); Compound (4) | Oxidation | m/z 189→205 |
| | Compound (5); Compound (6); Compound (7) | Hydrogenation (forming hydrocarbyl group) | m/z 189→207 |
| | Compound (8) | Tri-Demethylation | m/z 189→147 |
| | Compound (9) | +Keto (Oₓ-2H) or methylation | m/z 189→203 |

Table 2 shows the types of metabolites obtained from the reaction of the mixture of BP and cryopreserved hepatocytes (i.e., phase II metabolism) and the biotransformation pathway acquired by software analysis. The results show that compounds (11) to (14) of the present invention can be produced by the reaction of a mixture of BP and the cryopreserved hepatocytes of rat, dog or human, which indicates that BP can be transformed into similar metabolites when metabolized in the livers of different organisms.

**Table 2**

| Phase II metabolites | | | |
|---|---|---|---|
| Species | Metabolites | Biotransformation pathway | Mass shift |
| Rat | Compound (11) | + Cysteine | m/z 189→310 |
| | Compound (10) | + S-Glutathione | m/z 189→496 |
| | Compound (12) | Dehydrogenation+ Sulfonation | m/z 189→267 |
| | Compound (13) | Glucoronidation | m/z 189→365 |
| Dog | Compound (11) | + Cysteine | m/z 189→310 |
| | Compound (10) | + S-Glutathione | m/z 189→496 |
| | Compound (13) | Glucoronidation | m/z 189→365 |
| | Compound (14) | Dehydrogenation+ Oxidation+ Glucose | m/z 189→365 |
| Human | Compound (11) | + Cysteine | m/z 189→310 |
| | Compound (10) | + S-Glutathione | m/z 189→496 |
| | Compound (12) | Dehydrogenation+ Sulfonation | m/z 189→267 |
| | Compound (13) | + Glucoronidation | m/z 189→365 |

### [Example 2] In vivo assay: the survival rate of transgenic mice

It has been known that about 20% of ALS patients were associated with mutations in the gene that encodes Cu/Zn superoxide dismutase enzyme (SOD1), and G93A was the major mutation site. The mice transfected with human mutant SOD1-G93A by gene transfection technique (hereinafter referred to as "SOD1-G93A transgenic mice") was used as an animal model for the clinical study of ALS since the mice exhibit a similar course of disease to human. A SOD1-G93A transgenic mouse will show the symptoms of ALS within about 90 ± 5 days postnatal and will die within about 125 ± 5 days postnatal.

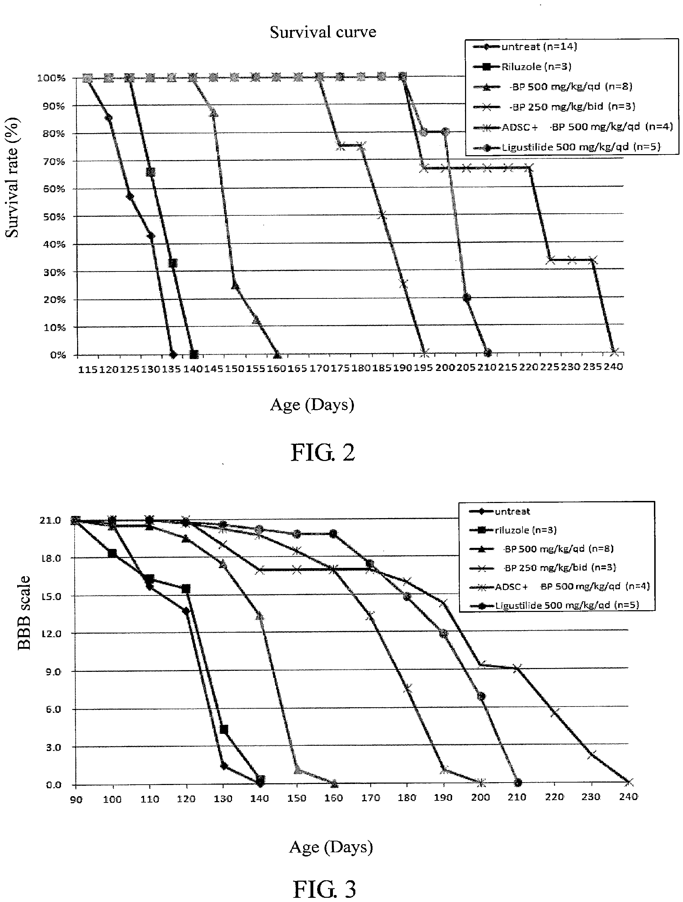

This example used the above SOD1-G93A transgenic mice as the object of study to perform *in vivo* assay. The mice were randomly distributed into the following six groups: (A) control group (untreated); (B) Riluzole-treated group: the SOD1-G93A transgenic mice at 60 days postnatal were treated with Riluzole at a dosage of 16 mg/kg-body weight once daily via intraperitoneal injection; (C) BP-treated group (BP 500 mg/kg/qd): the SOD1-G93A transgenic mice at 60 days postnatal were treated with BP (comprising Z-BP 95% + E-BP 5%; purchased from ECHO Chemical) at a dosage of 500 mg/kg-body weight once daily via oral administration; (D) BP-treated group (BP 250 mg/kg/bid): the SOD1-G93A transgenic mice at 60 days postnatal were treated with BP (comprising Z-BP 95% + E-BP 5%; purchased from ECHO Chemical) at a dosage of 250 mg/kg-body weight twice daily via oral administration; (E) combined treated group (ADSCs + BP): the SOD1-G93A transgenic mice were treated with BP (i.e., comprising Z-BP 95% + E-BP 5%; purchased from ECHO Chemical) at a dosage of 500 mg/kg-body weight once daily via oral administration at 60 days postnatal, transplanted with ADSCs (2x10⁶ cells/30 µl PBS) via intracerebral injection once at 60 days postnatal, and then transplanted with ADSCs (1x10⁶ cells/150 µl PBS) via intravenous injection at 90 days postnatal; (F) ligustilide-treated group: the SOD1-G93A transgenic mice at 60 days postnatal were treated with ligustilide (i.e., 3-butylidene-4,5-dihydrophthalide: a metabolic precursor of BP; purchased from Pharmaron) at a dosage of 500 mg/kg-body weight once daily via oral administration. After the SOD1-G93A transgenic mice were treated for 30 days, they were observed to see if a combined use of BP and ADSCs can prolong the longevity of the SOD1-G93A transgenic mice (i.e., more than 125 days). The results are shown in Figure 2 and Table 3.

**Table 3**

| Group | Survival days |
|---|---|
| control group (n = 14) | 126.4 ± 7.2 |
| Riluzole-treated group (n = 3) | 133.7 ± 6.4 |
| BP-treated group (BP 500 mg/kg/qd) (n= 8) | 149.1 ± 4.4 |
| BP-treated group (n-BP 250 mg/kg/bid) (n = 3) | 217.7 ± 23.2 |
| combined treated group (ADSC+BP) (n = 4) | 185 ± 7.5 |
| ligustilide-treated group (n = 5) | 201_{.}2 ± 6.0 |

As shown in Figure 2 and Table 3, as compared to the longevity of the SOD1-G93A transgenic mice in the control group (survived for about 126.4 ± 7.2), the longevity of the SOD1-G93A transgenic mice in the Riluzole-treated group (survived for about 133.7 ± 6.4) was merely prolonged for about 7.3± 0.8 days; the longevity of the SOD1-G93A transgenic mice in the BP-treated group (n-BP 250 mg/kg/bid) (survived for about 217.7 ± 23.2) was prolonged for about 91.3±16 days; the longevity of the SOD1-G93A transgenic mice in the ligustilide-treated group (survived for about 201.2 ± 6.0) was prolonged for about 74.8 ± 1.2 days; the longevity of the SOD1-G93A transgenic mice in the combined treated group (survived for about 185 ± 7.5) was prolonged for about 58.6 ± 0.3 days.

The above results show that as compared with the use of Riluzole or BP alone once daily via oral administration, the use of BP in combination with ADSCs of the present invention can more effectively increase the survival rate of the mice suffering from ALS. Furthermore, the use of ligustilide (i.e., a metabolic precursor of a phthalide) or BP alone twice daily via oral administration can effectively increase the survival rate of the mice suffering from ALS, and the increased survival rate is not only much higher than that of the use of BP alone once daily via oral administration, but also higher than that of the use of a combination of BP ADSCs.

### [Example 31 In vivo assay: amyotrophic lateral sclerosis

The above SOD1-G93A transgenic mice was used as the object of study to perform *in vivo* analysis in this example. The 60-days old SOD1-G93A transgenic mice were randomly distributed into the following six groups: (A) control group (untreated); (B) Riluzole-treated group: the SOD1-G93A transgenic mice at 60 days postnatal were treated with Riluzole at a dosage of 16 mg/kg-body weight once daily via intraperitoneal injection; (C) BP-treated group (BP 500 mg/kg/qd): the SOD1-G93A transgenic mice at 60 days postnatal were treated with BP (comprising Z-BP 95% + E-BP 5%; purchased from ECHO Chemical) at a dosage of 500 mg/kg-body weight once daily via oral administration; (D) BP-treated group (BP 250 mg/kg/bid): the SOD1-G93A transgenic mice at 60 days postnatal were treated with BP (comprising Z-BP 95% + E-BP 5%; purchased from ECHO Chemical) at a dosage of 250 mg/kg-body weight twice daily via oral administration; (E) combined treated group (ADSCs + BP): the SOD1-G93A transgenic mice were treated with BP (comprising Z-BP 95% + E-BP 5%; purchased from ECHO Chemical) at a dosage of 500 mg/kg-body weight once daily via oral administration at 60 days postnatal, transplanted with ADSCs (2x10⁶ cells/30 µl PBS) via intracerebral injection once at 60 days postnatal, and then transplanted with ADSCs (1x10⁶ cells/150 µl PBS) via intravenous injection at 90 days postnatal; (F) ligustilide-treated group: the SOD1-G93A transgenic mice at 60 days postnatal were treated with ligustilide (i.e., 3-butylidene-4,5-dihydrophthalide: a metabolic precursor of BP; purchased from Pharmaron) at a dosage of 500 mg/kg-body weight once daily via oral administration.

After the mice were treated for 30 days, the hind limbs of the mice were examined by BBB scale (Basso, Beattie, and Bresnahan (BBB) Locomotor Rating Scale). The BBB scale of the hind limbs of normal mice was 21 points, while the BBB scale of disease-progressed SOD1-G93A transgenic mice decreased from 21 to 0 points, wherein the lower scale represents a more severe motor disorder in the mice. BBB scale is used to record the efficiency of the treatments.

As shown in Figure 3, the BBB scale of the hind limbs of the untreated mice in the control group decreased rapidly after about 110 days (from 19 to 0 points) postnatal. The BBB scale of the hind limbs of the mice in the Riluzole-treated group decreased slowly from about 90 to 125 days (from 21 to 16 points), and decreased rapidly after about 125 days (from 16 to 0 points). The BBB scale of the hind limbs of the mice in the BP-treated group (BP 500 mg/kg/qd) decreased slowly from about 125 to 135 days (from 21 to 16 points), and decreased rapidly after about 135 days (from 16 to 0 points). The BBB scale of the hind limbs of the mice in the ligustilide-treated group decreased slowly from about 130 to 170 days (from 21 to 18 points), and decreased rapidly after about 170 days (from 18 to 0 points). The BBB scale of the hind limbs of the mice in the combined treated group (ADSCs + BP) decreased slowly from about 150 to 190 days postnatal (from 21 to 0 points).

The above results show that as compared with the use of Riluzole or BP alone once daily via oral administration, the use of BP in combination with ADSCs of the present invention can more effectively delay the onset of ALS. In addition, the use of ligustilide can effectively delay the onset of ALS as well, and the efficacy of delaying the onset of ALS is not only much superior to the use of BP alone once daily via oral administration, but also superior to the use of BP in combination with ADSCs.

### [Example 41 In vitro study: increase the telomerase expression level of ADSCs

ADSCs were isolated from human adipose tissue by the following steps. Human adipose tissue (obtained from clinical researches) was washed with phosphate-buffered saline (PBS) and minced with fine scissors. Then, the tissue was digested with 0.075% collagenase type I (Sigma-Aldrich Co., St. Louis, MO, USA) at 37°C for 30 to 60 minutes, and then Dulbecco's modified Eagle's medium (DMEM)/F-12 (Invitrogen, Carlsbad, CA, USA) containing 10% fetal bovine serum (FBS, Invitrogen) was added to terminate the digestion. The digested adipose tissue cells were filtered through a 100 µm nylon mesh to remove the cellular debris. The cell suspension was centrifuged for 10 minutes to obtain a precipitate, and then the precipitate was cultured at 37°C, 5% CO₂ in a DMEM/F-12 culture media (containing 10% FBS, 100 U/mL penicillin, and 100 mg/mL streptomycin). Then, the cultured cells were washed with PBS to remove the residual non-adherent red blood cells. The resultant cells (i.e., the stromal vascular fraction (SVF) containing ADSCs) were then cultured at 37°C, 5% CO₂ in a 10% FBS-containing DMEM/F-12 culture media. The adherent cells were maintained in the culture media, and the media were changed every 2 days. Upon reaching an 80% confluence, the cells were digested with 0.25% trypsin/ethylenediamine tetra-acetic acid (EDTA) at 37°C, centrifuged, and then re-suspended in the DMEM/F-12 culture media. Then, the cell suspensions were plated in a new flask and kept being cultured. After being sub-cultured for 3 times, the resultant cells were used for the following analysis.

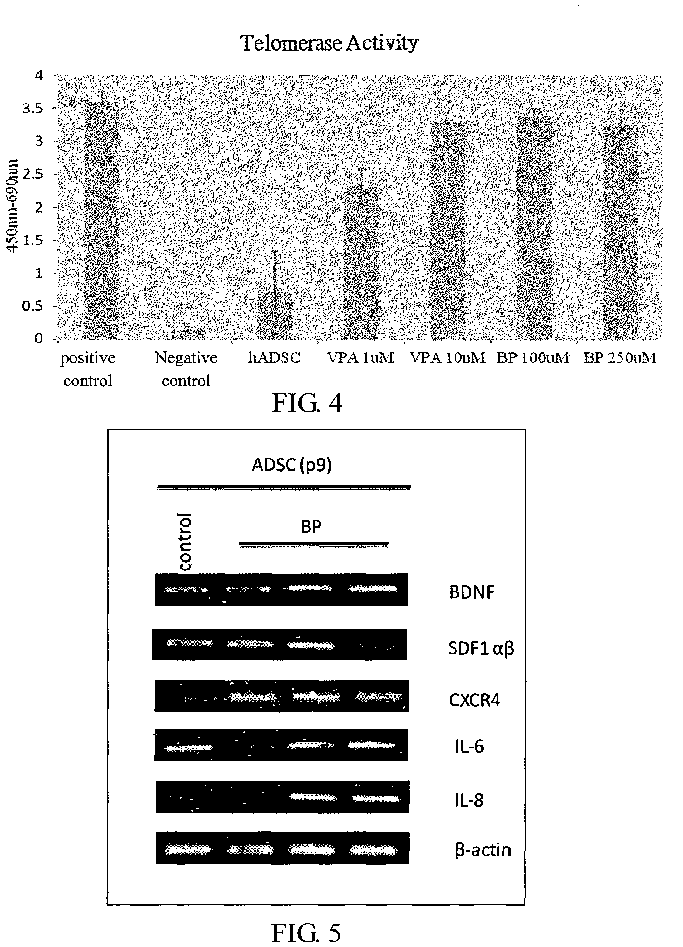

The ADSCs were cultured in a 10-cm culture dish, and treated with different concentrations of valproic acid (VPA) (1 µM or 10 µM) or BP (comprising Z-BP 95% + E-BP 5%) (100 µM or 250 µM) for 24 hours. Then, the telomerase expression level of the ADSCs was determined by a Telomerase PCR ELISA kit (Cat. No. 11854666910, Roche). In this experiment, the ADSC group was referred to the group does not be treated with BP; the positive control group was referred to the sample provided by the ELISA kit which has a high expression level of telomerase (a reconstitute lyophilized cell extract); the negative control group was referred to the sample provided by the ELISA kit which has a high expression level of telomerase whose protein activity was inactivated via being heated at 85°C for 10 minutes. In addition, previous researches showed that VPA can be used to increase the telomerase activity of ADSCs, and thus, VPA was used in this experiment as a control group. The results are shown in Figure 4.

As shown in Figure 4, when the ADSCs treated with BP (100 µM or 250 µM), the telomerase activity of the ADSCs can be increased effectively (i.e., the expression level of telomerase of the ADSCs can be increased). The above results show that BP can increase the expression level of telomerase of ADSCs, thereby prolonging the lifespan of ADSCs.

### [Example 5] In vitro study: increasing the expression levels of brain-derived neurotrophic factor, stromal cell-derived factor-1, CXC chemokine receptor 4, and immune regulatory factors of ADSCs

Human ADSCs (2x10⁵ cells/well) were cultured in a 6-well culture dish at 37°C overnight. Then, the ADSCs were added with BP (comprising 95% Z-BP + 5% E-BP) of different concentrations (10 µg/ml, 20 (µg/ml, or 50 µg/ml) and incubated for 12 hours. The gene expression levels of brain-derived neurotrophic factor (BDNF), stromal cell-derived factor-1 (SDF1), C-X-C chemokine receptor type 4 (CXCR4), interleukin-6 (IL-6), and interleukin-8 (IL-8) of the ADSCs were determined by RT-PCR. The sequences of the primers used in the RT-PCR are SEQ ID NO: 1 to SEQ ID NO: 10 as shown in Table 4 and the accompanying sequence listing. β-actin was used as a control group in the experiment. The results are shown in Figure 5.

**Table 4**

| Primer | SEQ ID NO | Sequence (5' to 3') |
|---|---|---|
| BDNF primer- Forward Sequence | SEQ ID NO: 1 | gtgtgcgaca gcattagcca gtgg |
| BDNF primer- Reverse Sequence | SEQ ID NO: 2 | cacatacatg aaactggtaa ttctcc |
| SDF1 primer- Forward Sequence | SEQ ID NO: 3 | atgaacgcca aggtcgtggt c |
| SDF1 primer- Reverse Sequence | SEQ ID NO: 4 | tcatggacct ctttcgaaat ttgttc |
| CXCR4 primer-Forward Sequence | SEQ ID NO: 5 | ggccctcaag accacagtca |
| CXCR4 primer- Reverse Sequence | SEQ ID NO: 6 | gaagttcaaa agtgaggtcg att |
| interleukin-6 primer- Forward Sequence | SEQ ID NO: 7 | tgccagcctg ctgacgaagc |
| interleukin-6 primer- Reverse Sequence | SEQ ID NO: 8 | tctgtgccca gtggacaggt |
| interleukin-8 primer- Forward Sequence | SEQ ID NO: 9 | gctggccgtg gctctcttgg |
| interleukin-8 primer- Reverse Sequence | SEQ ID NO: 10 | tccacaaccc tctgcaccca |

As shown in Figure 5, after the ADSCs were treated with BP (especially at the concentration of 20 µg/ml) for 12 hours, the gene expression levels of BDNF, SDF1, CXCR4 were increased. In addition, after the ADSCs were treated with BP, the gene expression levels of IL-6 and IL-8 were also increased, indicating that BP can increase the gene expression levels of cytokines to regulate the immune response, and thereby maintaining neurogenesis. The above results show that the use of BP in combination with ADSCs can increase the expression levels of BDNF, SDF 1, CXCR4, IL-6, and IL-8 of the ADSCs, and thus, can inhibit the apoptosis of neurons and promote the proliferation of neurons.

### [Example 6] In vivo study: increasing the protein expression levels of brain-derived neurotrophic factor and CXC chemokine receptor 4 of ADSCs

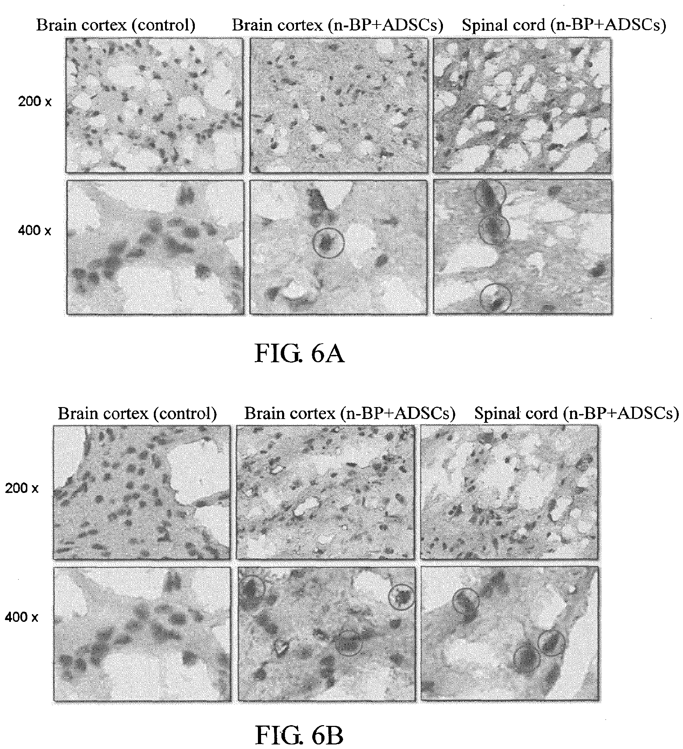

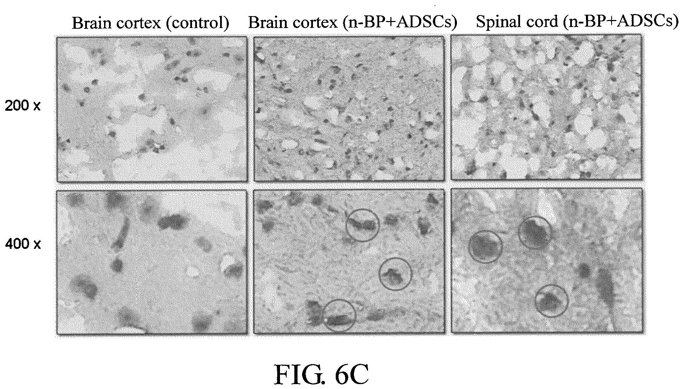

SOD1-G93A transgenic mice were treated with BP (comprising Z-BP 95% + E-BP 5%) at a dosage of 500 mg/kg-body weight once daily via oral administration at 60 days postnatal, transplanted with ADSCs (2x10⁶ cells/30µl PBS) via intracerebral injection once at 60 days postnatal, and then transplanted with ADSCs (1x10⁶ cells/150 µl PBS) via intravenous injection once at 90 days postnatal. The mice were anesthetized with chloral hydrate and sacrificed at 90 days postnatal. The spinal cords and the brain tissues of the mice were excised for immunohistochemistry (IHC) analysis. The excised spinal cords and the brain tissues were postfixed overnight in 4% paraformaldehyde and subsequently cryoprotected in 30% sucrose and sectioned with a Leica cryostat to a thickness of 10 µm. Serial sections were cut from the spinal cord, frontal cortex or hippocampus of the mice, and mounted on gelatin-coated slides. The slides were incubated in a blocking solution, and then were overnight incubated at 4°C with an anti-human mitochondria antibody (purchased from Abcan) (which can be used to identify the injected ADSCs), an anti-human BDNF antibody (purchased from GeneTex), or an anti-human CXCR4 antibody (purchased from GeneTex). The results are shown in Figures 6A, 6B and 6C.

As shown in Figure 6A, human ADSCs (see the positions marked with circles in Figure 6A) can be detected in the spinal cord of the mice which have been treated with BP in combination with ADSCs. This result demonstrates that the intracerebral injected (or intravenous injected) ADSCs indeed can migrate from the brain (or the vein) to the spinal cord, and thereby can protect the spinal motor neurons. In addition, as shown in Figures 6B and 6C, the levels of human BDNF (see the positions marked with circles in Figure 6B) and human CXCR4 (see the positions marked with circles in Figure 6C) detected in the brain and spinal cord of the mice were significantly increased after the SOD1-G93A transgenic mice were treated with BP in combination with ADSCs.

The above results show that the use of BP in combination with ADSCs can increase the protein expression levels of BDNF and CXCR4 of ADSCs, and thus, can inhibit the apoptosis of neurons and promote the proliferation of neurons.

The results in the above examples show that a use of a phthalide in combination with a stem cell can increase the expression levels of telomerase, brain-derived neurotrophic factor, stromal cell-derived factor-1, CXC chemokine receptor 4, and immune regulatory factors (e.g., IL-6 and IL-8) in a stem cell of a subject, and thus, can inhibit the apoptosis of motor neurons, protect motor neurons, and/or promote the proliferation of motor neurons in the subject, and can treat motor neuron degenerative diseases and/or delay the onset of motor neuron degenerative diseases.

The above examples are used to illustrate the principle and efficacy of the present invention but not used to limit to the present invention. People skilled in this field may proceed with a variety of modifications and replacements based on the disclosures and suggestions of the invention as defined in the claims as appended.

## Claims

1. A pharmaceutical composition comprising a phthalide for use as a medicament for inhibiting the apoptosis of motor neurons, protecting motor neurons, and/or promoting the proliferation of motor neurons by increasing the expression level of at least one of telomerase, brain-derived neurotrophic factor (BDNF), stromal cell-derived factor-1 (SDF1), CXC chemokine receptor 4 (CXCR4), IL-6 and IL-8 in a stem cell, wherein the phthalide and a stem cell are administered to a subject simultaneously or separately, and the phthalide is selected from the following group: n-butylidenephthalide (BP), 3-butylidene-4,5-dihydrophthalide (ligustilide), and combinations thereof.

2. The pharmaceutical composition for use as claimed in Claim 1, wherein the phthalide is n-butylidenephthalide.

3. The pharmaceutical composition for use as claimed in Claim 1 or 2, wherein the stem cell is selected from the group consisting of an embryonic stem cell, an adult stem cell, an induced pluripotent stem cell, and combinations thereof.

4. The pharmaceutical composition for use as claimed in Claim 1 or 2, wherein the stem cell is selected from the group consisting of a mesenchymal stem cell, a hematopoietic stem cell, and combinations thereof.

5. The pharmaceutical composition for use as claimed in Claim 1 or 2, wherein the stem cell is a mesenchymal stem cell selected from the group consisting of a bone marrow stem cell, an umbilical cord blood stem cell, a placenta stem cell, an adipose stem cell, an oral stem cell, an olfactory bulbs stem cell, an amniotic fluid stem cell, an amniotic stem cell, an umbilical cord stem cell, an umbilical cord lining stem cell, and combinations thereof.

6. The pharmaceutical composition for use as claimed in Claim 1 or 2, wherein the phthalide and a stem cell are administered to a subject simultaneously or separately, to treat a motor neuron degenerative disease and/or delay the onset of a motor neuron degenerative disease.

7. The pharmaceutical composition for use as claimed in Claim 6, wherein the motor neuron degenerative disease is selected from the group consisting of amyotrophic lateral sclerosis, myasthenia gravis, myasthenia, muscular atrophy, muscular dystrophy, multiple sclerosis, multiple system atrophy, spinal muscular atrophy, and combinations thereof.

8. The pharmaceutical composition for use as claimed in Claim 6, wherein the motor neuron degenerative disease is amyotrophic lateral sclerosis.

9. A kit for use as a medicament, comprising:
a first composition, comprising a phthalide; and
a second composition, comprising a stem cell;
wherein the first composition and the second composition are to be administered to a subject in need simultaneously or separately; and wherein the medicament is selected from the following group: n-butylidenephthalide (BP), 3-butylidene-4,5-dihydrophthalide (ligustilide), and combinations thereof.

10. The kit as claimed in Claim 9, wherein the phthalide is n-butylidenephthalide.

11. The kit as claimed in Claim 9 or 10, wherein the stem cell is selected from the group consisting of an embryonic stem cell, an adult stem cell, an induced pluripotent stem cell, and combinations thereof.

12. The kit as claimed in Claim 9 or 10, wherein the stem cell is selected from the group consisting of a mesenchymal stem cell, a hematopoietic stem cell, and combinations thereof.

13. The kit as claimed in Claims 9 to 12, wherein the stem cell is a mesenchymal stem cell selected from the following group: a bone marrow stem cell, an umbilical cord blood stem cell, a placenta stem cell, an adipose stem cell, an oral stem cell, an olfactory bulbs stem cell, an amniotic fluid stem cell, an amniotic stem cell, an umbilical cord stem cell, an umbilical cord lining stem cell, and combinations thereof.

14. The kit as claimed in any one of Claims 9 to 13, further comprises a third composition which comprises a solvent or a solution and can be mixed with the first composition and/or the second composition to provide a solution for injection.

15. The kit as claimed in any one of Claims 9 to 14, wherein the first composition is in a form for oral administration, nasal administration, corticospinal tract injection, intrathecal injection, intracerebral injection, intravenous injection, intraperitoneal injection, and/or subcutaneous injection; and the second composition is in a form for corticospinal tract injection, intrathecal injection, intracerebral injection, intravenous injection, intraperitoneal injection, and/or subcutaneous injection.

## Patentansprüche

1. Pharmazeutische Zusammensetzung, die ein Phthalid umfasst, zur Verwendung als Medikament zur Hemmung der Apoptose von motorischen Neuronen, zum Schutz von motorischen Neuronen und/oder zur Förderung der Vermehrung von motorischen Neuronen durch Erhöhung des Expressionsniveaus von wenigstens einem aus Telomerase, dem neurotrophen Wachstumsfaktor BDNF, SDF1, CXC-Chemokin-Rezeptor 4 (CXCR4), IL-6 und IL-8 in einer Stammzelle, wobei das Phthalid und eine Stammzelle gleichzeitig oder getrennt an einen Patienten verabreicht werden und das Phthalid aus der folgenden Gruppe ausgewählt ist: n-Butylidenphthalid (BP), 3-Butyliden-4,5-dihydrophthalid (Ligustilid) und Kombinationen davon.

2. Pharmazeutische Zusammensetzung zur Verwendung gemäß Anspruch 1, wobei es sich bei dem Phthalid um n-Butylidenphthalid handelt.

3. Pharmazeutische Zusammensetzung zur Verwendung gemäß Anspruch 1 oder 2, wobei die Stammzelle aus der Gruppe ausgewählt ist, die aus einer embryonalen Stammzelle, einer adulten Stammzelle, einer induzierten pluripotenten Stammzelle und Kombinationen davon besteht.

4. Pharmazeutische Zusammensetzung zur Verwendung gemäß Anspruch 1 oder 2, wobei die Stammzelle aus der Gruppe ausgewählt ist, die aus einer mesenchymalen Stammzelle, einer hämatopoetischen Stammzelle und Kombinationen davon besteht.

5. Pharmazeutische Zusammensetzung zur Verwendung gemäß Anspruch 1 oder 2, wobei die Stammzelle eine mesenchymale Stammzelle ist, die aus der Gruppe ausgewählt ist, die aus einer Knochenmark-Stammzelle, einer Nabelschnurblut-Stammzelle, einer Plazenta-Stammzelle, einer Fettgewebe-Stammzelle, einer oralen Stammzelle, einer Riechkolben-Stammzelle, einer Fruchtwasser-Stammzelle, einer amniotischen Stammzelle, einer Nabelschnur-Stammzelle, einer Nabelschnurwand-Stammzelle und Kombinationen davon besteht.

6. Pharmazeutische Zusammensetzung zur Verwendung gemäß Anspruch 1 oder 2, wobei das Phthalid und eine Stammzelle gleichzeitig oder getrennt an einen Patienten verabreicht werden, zur Behandlung einer degenerativen Erkrankung der motorischen Neuronen und/oder zur Verzögerung des Einsetzens einer degenerativen Erkrankung der motorischen Neuronen.

7. Pharmazeutische Zusammensetzung zur Verwendung gemäß Anspruch 6, wobei die degenerative Erkrankung der motorischen Neuronen aus der Gruppe ausgewählt ist, die aus amyotropher Lateralsklerose, Myasthenia gravis, Myasthenie, Muskelatrophie, Muskeldystrophie, multipler Sklerose, multipler Systematrophie, spinaler Muskelatrophie und Kombinationen davon besteht.

8. Pharmazeutische Zusammensetzung zur Verwendung gemäß Anspruch 6, wobei es sich bei der degenerativen Erkrankung der motorischen Neuronen um amyotrophe Lateralsklerose handelt.

9. Kit zur Verwendung als Medikament, umfassend:
ein erste Zusammensetzung, die ein Phthalid umfasst; und
ein zweite Zusammensetzung, die eine Stammzelle umfasst;
wobei die erste Zusammensetzung und die zweite Zusammensetzung gleichzeitig oder getrennt an einen Patienten, der diese benötigt, verabreicht werden sollen und wobei das Medikament aus der folgenden Gruppe ausgewählt ist: n-Butylidenphthalid (BP), 3-Butyliden-4,5-dihydrophthalid (Ligustilid) und Kombinationen davon.

10. Kit gemäß Anspruch 9, wobei es sich bei dem Phthalid um n-Butylidenphthalid handelt.

11. Kit gemäß Anspruch 9 oder 10, wobei die Stammzelle aus der Gruppe ausgewählt ist, die aus einer embryonalen Stammzelle, einer adulten Stammzelle, einer induzierten pluripotenten Stammzelle und Kombinationen davon besteht.

12. Kit gemäß Anspruch 9 oder 10, wobei die Stammzelle aus der Gruppe ausgewählt ist, die aus einer mesenchymalen Stammzelle, einer hämatopoetischen Stammzelle und Kombinationen davon besteht.

13. Kit gemäß Anspruch 9 bis 12, wobei die Stammzelle eine mesenchymale Stammzelle ist, die aus der Gruppe ausgewählt ist, die aus einer Knochenmark-Stammzelle, einer Nabelschnurblut-Stammzelle, einer Plazenta-Stammzelle, einer Fettgewebe-Stammzelle, einer oralen Stammzelle, einer Riechkolben-Stammzelle, einer Fruchtwasser-Stammzelle, einer amniotischen Stammzelle, einer Nabelschnur-Stammzelle, einer Nabelschnurwand-Stammzelle und Kombinationen davon besteht.

14. Kit gemäß einem der Ansprüche 9 bis 13, der weiterhin eine dritte Zusammensetzung umfasst, die ein Lösungsmittel oder eine Lösung umfasst und mit der ersten Zusammensetzung und/oder der zweiten Zusammensetzung gemischt werden kann, um eine Lösung für die Injektion zu erhalten.

15. Kit gemäß einem der Ansprüche 9 bis 14, wobei die erste Zusammensetzung in einer Form für die orale Verabreichung, nasale Verabreichung, Pyramidenbahn-Injektion, intrathekale Injektion, intrazerebrale Injektion, intravenöse Injektion, intraperitoneale Injektion und/oder subkutane Injektion vorliegt und die zweite Zusammensetzung in einer Form für die Pyramidenbahn-Injektion, intrathekale Injektion, intrazerebrale Injektion, intravenöse Injektion, intraperitoneale Injektion und/oder subkutane Injektion vorliegt.

## Revendications

1. Composition pharmaceutique comprenant un phtalide pour utilisation comme médicament pour inhiber l'apoptose des motoneurones, protéger les motoneurones, et/ou favoriser la prolifération des motoneurones en augmentant le niveau d'expression d'au moins un parmi la télomérase, le facteur neurotrophique dérivé du cerveau (BDNF), le facteur-1 dérivé des cellules stromales (SDF1), le récepteur 4 de chimiokine CXC (CXCR4), IL-6 et IL-8 dans une cellule souche, dans laquelle le phtalide et une cellule souche sont administrés simultanément ou séquentiellement à un patient, et le phtalide est choisi dans le groupe suivant : n-butylidènephtalide (BP), 3-butylidène-4,5-dihydrophtalide (ligustilide), et des combinaisons de ceux-ci.

2. Composition pharmaceutique pour utilisation selon la revendication 1, dans laquelle ledit phtalide est le n-butylidènephtalide.

3. Composition pharmaceutique pour utilisation selon la revendication 1 ou 2, dans laquelle ladite cellule souche est choisie dans le groupe consistant en une cellule souche embryonnaire, une cellule souche adulte, cellule souche pluripotente induite, et des combinaisons de celles-ci.

4. Composition pharmaceutique pour utilisation selon la revendication 1 ou 2, dans laquelle ladite cellule souche est choisie dans le groupe consistant en une cellule souche mésenchymateuse, une cellule souche hématopoïétique, et des combinaisons de celles-ci.

5. Composition pharmaceutique pour utilisation selon la revendication 1 ou 2, dans laquelle ladite cellule souche est une cellule souche mésenchymateuse choisie dans le groupe consistant en une cellule souche de la moelle osseuse, une cellule souche du sang de cordon ombilical, une cellule souche du placenta, une cellule souche du tissu adipeux, une cellule souche orale, une cellule souche du bulbe olfactif, une cellule souche du liquide amniotique, une cellule souche amniotique, une cellule souche du cordon ombilical, une cellule souche issue de la membrane amniotique du cordon ombilical, et des combinaisons de celles-ci.

6. Composition pharmaceutique pour utilisation selon la revendication 1 ou 2, dans laquelle ledit phtalide et une cellule souche sont administrés simultanément ou séquentiellement à un patient pour traiter une maladie dégénérative des motoneurones, et/ou pour retarder l'apparition d'une maladie dégénérative des motoneurones.

7. Composition pharmaceutique pour utilisation selon la revendication 6, dans laquelle la maladie dégénérative des motoneurones est choisie dans le groupe consistant en sclérose latérale amyotrophique, myasthénie grave, myasthénie, atrophie musculaire, dystrophie musculaire, sclérose en plaques, atrophie systémique multiple, amyotrophie spinale, et des combinaisons de ceux-ci.

8. Composition pharmaceutique pour utilisation selon la revendication 6, dans laquelle la maladie dégénérative des motoneurones est la sclérose latérale amyotrophique.

9. Trousse pour utilisation comme médicament, comprenant :
une première composition comprenant un phtalide, et
une deuxième composition comprenant une cellule souche,
dans laquelle lesdites première et deuxième compositions doivent être administrées simultanément ou séquentiellement à un patient le nécessitant, et dans laquelle le médicament est choisi dans le groupe suivant : n-butylidènephtalide (BP), 3-butylidène-4,5-dihydrophtalide (ligustilide), et des combinaisons de ceux-ci.

10. Trousse selon la revendication 9, dans laquelle ledit phtalide est le n-butylidènephtalide.

11. Trousse selon la revendication 9 ou 10, dans laquelle ladite cellule souche est choisie dans le groupe consistant en une cellule souche embryonnaire, une cellule souche adulte, cellule souche pluripotente induite, et des combinaisons de celles-ci.

12. Trousse selon la revendication 9 ou 10, dans laquelle ladite cellule souche est choisie dans le groupe consistant en une cellule souche mésenchymateuse, une cellule souche hématopoïétique, et des combinaisons de celles-ci.

13. Trousse selon la revendication 9 à 12, dans laquelle ladite cellule souche est une cellule souche mésenchymateuse choisie dans le groupe consistant en une cellule souche de la moelle osseuse, une cellule souche du sang de cordon ombilical, une cellule souche du placenta, une cellule souche du tissu adipeux, une cellule souche orale, une cellule souche du bulbe olfactif, une cellule souche du liquide amniotique, une cellule souche amniotique, une cellule souche du cordon ombilical, une cellule souche issue de la membrane amniotique du cordon ombilical, et des combinaisons de celles-ci.

14. Trousse selon l'une quelconque des revendications 9 à 13, comprenant en outre une troisième composition qui comprend un solvant ou une solution et peut être mélangée avec la première composition et/ou la deuxième composition pour procurer une solution pour injection.

15. Trousse selon l'une quelconque des revendications 9 à 14, dans laquelle la première composition est dans une forme pour l'administration orale, l'administration nasale, l'injection dans la voie corticospinale, l'injection intrathécale, l'injection intracérébrale, l'injection intraveneuse, l'injection intrapéritonéale, et/ou l'injection souscutanée, et la deuxième composition est dans une forme pour l'injection dans la voie corticospinale, l'injection intrathécale, l'injection intracérébrale, l'injection intraveneuse, l'injection intrapéritonéale, et/ou l'injection souscutanée.


### SEQUENCE LISTING

<110> HAWKING BIOLOGICAL TECHNOLOGY CO., LTD
<120> APPLICATION OF PHTHALIDE COMPOUND
<130> 03850PCT-EP
<140> PCT/CN2014/000155
   <141> 2014-02-13
<150> US 61/777,127
   <151> 2013-03-12
<160> 10
<170> PatentIn version 3.5
<210> 1
   <211> 24
   <212> DNA
   <213> Artificial Sequence
<220>
   <223> BDNF primer- Forward Sequence
<400> 1
   gtgtgcgaca gcattagcca gtgg 24
<210> 2
   <211> 26
   <212> DNA
   <213> Artificial Sequence
<220>
   <223> BDNF primer- Reverse Sequence
<400> 2
   cacatacatg aaactggtaa ttctcc 26
<210> 3
   <211> 21
   <212> DNA
   <213> Artificial Sequence
<220>
   <223> SDF1 primer- Forward Sequence
<400> 3
   atgaacgcca aggtcgtggt 21
<210> 4
   <211> 26
   <212> DNA
   <213> Artificial Sequence
<220>
   <223> SDF1 primer- Reverse Sequence
<400> 4
   tcatggacct ctttcgaaat ttgttc 26
<210> 5
   <211> 20
   <212> DNA
   <213> Artificial Sequence
<220>
   <223> CXCR4 primer- Forward Sequence
<400> 5
   ggccctcaag accacagtca 20
<210> 6
   <211> 23
   <212> DNA
   <213> Artificial Sequence
<220>
   <223> CXCR4 primer- Reverse Sequence
<400> 6
   gaagttcaaa agtgaggtcg att 23
<210> 7
   <211> 20
   <212> DNA
   <213> Artificial Sequence
<220>
   <223> interleukin 6 primer- Forward Sequence
<400> 7
   tgccagcctg ctgacgaagc 20
<210> 8
   <211> 20
   <212> DNA
   <213> Artificial Sequence
<220>
   <223> interleukin 6 primer- Reverse Sequence
<400> 8
   tctgtgccca gtggacaggt 20
<210> 9
   <211> 20
   <212> DNA
   <213> Artificial Sequence
<220>
   <223> interleukin 8 primer- Forward Sequence
<400> 9
   gctggccgtg gctctcttgg 20
<210> 10
   <211> 20
   <212> DNA
   <213> Artificial Sequence
<220>
   <223> interleukin 8 primer- Reverse Sequence
<400> 10
   tccacaaccc tctgcaccca 20